# EUROPÄISCHE PATENTSCHRIFT

(11) **EP 1 075 253 B1**
(45) Veröffentlichungstag und Bekanntmachung des Hinweises auf die Patenterteilung: **30.06.2004**
(21) Anmeldenummer: 99923543.5
(22) Anmeldetag: 06.05.1999
(51) Int. Cl.: A61K 9/26, A61K 9/02

(54) **WIRKSTOFF ENTHALTENDE ORALE ODER MUCOSALE ZUBEREITUNG MIT STEUERBARER WIRKSTOFFFREISETZUNG UND IHRE VERWENDUNG**
ORAL OR MUCOSAL PREPARATION CONTAINING AN ACTIVE INGREDIENT, WITH CONTROLLED ACTIVE INGREDIENT RELEASE, AND ITS USE
PREPARATION ORALE OU MUCOSIQUE CONTENANT UN PRINCIPE ACTIF, A LIBERATION REGULABLE DU PRINCIPE ACTIF ET SON UTILISATION

(30) Priorität: 08.05.1998 DE 19820529
(43) Veröffentlichungstag der Anmeldung: 14.02.2001
(73) Patentinhaber: LTS Lohmann Therapie-Systeme AG, 56626 Andernach (DE)
(72) Erfinder: VON FALKENHAUSEN, Christian, D-53125 Bonn (DE)
(74) Vertreter: Flaccus, Rolf-Dieter, Dr.
(86) Internationale Anmeldenummer: PCT/EP1999/003110
(87) Internationale Veröffentlichungsnummer: WO 1999/058107

(56) Entgegenhaltungen:
- EP-A- 0 636 365
- WO-A-94/28911
- US-A- 2 996 431

## Beschreibung

Die Erfindung betrifft eine Wirkstoff enthaltende orale rektale oder vaginale Zubereitung zur transmucosalen Resorption des Wirkstoffs mit steuerbarer Wirkstofffreisetzungsrate und ihre Verwendung.

Orale Darreichungsformen geben ihren Wirkstoff in der Regel durch Diffusion oder Zerfall ab, woraus eine nicht-lineare Freisetzungskinetik resultiert. Eine häufige Anforderung an orale pharmazeutische Systeme stellt die lineare Wirkstofffreisetzung nullter Ordnung aus der Applikationsform dar. Darüber hinaus kann es wünschenswert sein, das Freisetzungsprofil entsprechend spezifischen Anforderungen beliebig modulieren zu können. Systeme, welche den gewünschten Effekt erzielen, sind üblicherweise kompliziert und teuer in der Herstellung.

Das Dokument US 4,606,909 beschreibt eine pharmazeutische Zubereitung zur oralen Applikation mit einem homogenen Kern in einer inneren Umhüllung, die bei pH-Wert unter 7,5 unlöslich, dagegen im Dünndarmbereich löslich ist, wobei das Material der Umhüllung ausgewählt ist aus einer Gruppe von Acryl-Polymeren und jeder Kern aus einem multipartikulären System von Partikeln einer Durchschnittsgröße zwischen 1 und 10 µm besteht, und die Partikel eine geringfügig lösbare aktive Substanz enthalten.

In WO 85/03437 ist eine diffusionskontrollierte, multipartikuläre orale Formulierung mit wirkstoffhaltigen individuellen Einheiten beschrieben, die mit einer wasserunlöslichen, jedoch wasserdiffundierbaren Umhüllung aus einer Kombination eines wasserdispersiblen Filmbildners und einer Polymersubstanz zur Verhinderung eines Verklebens der Einheiten bei erhöhter Temperatur versehen sind, welche den Einheiten eine gewisse Fließfähigkeit verleiht. Die Umhüllung besitzt eine ausreichende plastische Verformbarkeit, die eine signifikante Veränderung im Freisetzungsverhalten des Wirkstoffes bei komprimierten oder nicht-komprimierten Einheiten verhindert.

US-A-2 996 431 beschreibt eine orale Zubereitung (Tablet) mit steuerbarer Wirkstofffreisetzung bestehend aus einem multipartikulären System der Partikel in einer Trägersubstanz.

DE 39 18 801 A1 beschreibt ein Präparat mit geregelter Wirkstoffabgabe, welches aus multipartikulären Formlingen mit wenigstens einem Wirkstoff und wenigstens einer die Geschwindigkeit der Wirkstofffreisetzung regelnden, inaktiven Komponente besteht, die zwei bis vier Schichten umfaßt. Dabei besteht die Erfindung darin, daß
a) die innerste Schicht aus einem inaktiven Kern besteht oder mit der nach außen folgenden identisch ist,
b) die nach außen hin folgende Schicht aus Wirkstoff und möglicherweise zusätzlich aus inaktiver Komponente besteht, die nach Art oder Menge nicht geeignet ist, die Wirkstofffreigabe wesentlich zu regeln,
c) die darauf folgende Schicht den höchsten Anteil an der inaktiven, die Geschwindigkeit der Freisetzung regelnden Komponente und zusätzlich feste Bestandteile nach Art einer Matrix enthält und die Geschwindigkeit regelt, und
d) möglicherweise eine weitere Schicht folgt, die die schnelle Freisetzung des Wirkstoffs zu Beginn regelt, aber keinen wesentlichen Einfluß auf die Geschwindigkeit der Freisetzung hat.

Der Erfindung liegt die Aufgabe zugrunde, eine Applikationsform für eine Wirkstoff enthaltende orale rektalen oder vaginalen Zubereitung zur transmucosalen Resorption des Wirkstoffs anzugeben, die weniger kompliziert ist und mit einfachen sowie kostengünstigen Mitteln eine beliebig modulierbare Freisetzungskinetik aufweist.

Die Lösung der Aufgabe gelingt bei einer irkstoff enthaltenden oralen rektalen oder vaginalen Zubereitung zur transmucosalen Resorption des Wirkstoffs mit steuerbarer Wirkstofffreisetzungsrate der im Oberbegriff von Anspruch 1 genannten Art durch eine Ausbildung entsprechend den im Anspruch 1 angegebenen Merkmalen.

Der Erläuterung der Erfindung dienen die folgenden Definitionen:
Zubereitung: Eine Zusammensetzung, enthaltend eine erste Gruppe von Partikeln und mindestens eine weitere Gruppe von Partikeln, die nicht identisch mit der ersten Gruppe von Partikeln ist.
Partikel: Darunter sind kugelförmige und nicht-kugelförmige Teilchen im Korngrößenbereich oberhalb von 10 µm zu verstehen. Grundsätzlich ist die Obergrenze der Partikelgröße offen, für den vorgesehenen Anwendungszweck im pharmazeutischen oder veterinärmedizinischen Bereich sind jedoch Obergrenzen von 3 mm bevorzugt. Die Partikel enthalten einen Kern und eine den Kern vollständig umschließende Umhüllung, die auch als Mantelschicht bezeichnet werden kann. In einer besonderen Ausführungsform kann der Kern der Partikel auch von mehr als einer Mantelschicht umhüllt sein.
Kern: Der Kern ist ein kugelförmiges oder nicht-kugelförmiges Teilchen im Korngrößenbereich oberhalb von 1 µm. Grundsätzlich ist die Obergrenze der Kerngröße offen, für den vorgesehenen Anwendungszweck im pharmazeutischen oder veterinärmedizinischen Bereich ist jedoch eine Obergrenze von 2 mm bevorzugt. Bevorzugt sind Kerne mit Kerngrößen zwischen 10 und 50 µm. Der Kern enthält eine Kernsubstanz und mindestens einen Wirkstoff.
Mantelschicht: Die Mantelschicht umhüllt den Kern der Partikel vollständig. Im Fall von kugelförmigen Kernen besitzt die Mantelschicht die Form einer Kugelschale. Die Mantelschicht bzw. Mantelschichten können ebenfalls grundsätzlich beliebig dick sein. Die Dicke der Mantelschicht entspricht dabei der Korngröße der Partikel abzüglich der Korngröße des Kerns geteilt durch 2. Im Fall von kugelförmigen Partikeln mit kugelförmigen Kernen entspricht die Dicke der Mantelschicht(en) dem Radius der Partikel abzüglich dem Radius des Kerns. Die Dicke der Mantelschicht ist grundsätzlich nicht beschränkt, das heißt, es existiert im Prinzip keine Obergrenze. Die Dicke der Mantelschicht ist aber für einen speziellen Kern stets im wesentlichen konstant. Für die vorgesehenen pharmazeutischen Zwecke sind jedoch Umhüllungsdicken zwischen 10 und 1000 µm bevorzugt. Besonders bevorzugt sind Umhüllungsdicken zwischen 10 und 100 µm. Die Mantelschicht enthält eine Mantelsubstanz und ist im wesentlichen frei von dem Wirkstoff, der im Kern enthalten ist.
Gruppe: Eine Vielzahl von identischen Partikeln bildet eine Gruppe von Partikeln. Das heißt, daß alle Partikel dieser Gruppe dieselbe einheitliche Kerngröße und dieselbe einheitliche Mantelschicht besitzen. Auch alle anderen Eigenschaften der Partikel, die zu einer Gruppe von Partikeln zählen, sind im wesentlichen gleich. Das hat zur Folge, daß eine einzelne Gruppe von Partikeln dementsprechend in einem bestimmten Medium ein charakteristisches Freisetzungsverhalten für den oder die Wirkstoffe zeigt.

Mit Vorteil wird bei Partikeln mit gleicher Kernsubstanz und gleicher Umhüllungssubstanz, wobei diese entsprechend den relativen Gewichtsanteilen von Kernsubstanz und Umhüllungssubstanz unterschiedlichen Gruppen zuzuordnen sind, und wobei gleiche Gruppen ein gleiches Erosions- und Freisetzungsverhalten aufweisen, von der sich daraus ergebenden Möglichkeit zur Steuerung der Freisetzungskinetik des Wirkstoffs Gebrauch gemacht. Diese Steuerung erfolgt dabei über das Mischungsverhältnis unterschiedlicher Gruppen von Partikeln. Beispielsweise können einzelne Gruppen ausgewählt sein, die sich untereinander durch stufenweise Verzögerung des Freisetzungsbeginns unterscheiden. Bei Zusammenstellung solcher Gruppen in der Art, daß sie beispielsweise in jeweils gleicher zeitlicher Abstufung mit der Freisetzung von Wirkstoff beginnen, kann ein beliebiger Wirkstofffreisetzungsverlauf gesteuert werden beziehungsweise das zeitliche Freisetzungsprofil entsprechend spezifischen Anforderungen beliebig moduliert werden. Damit erfüllt die erfindungsgemäße Zusammensetzung mit steuerbarer Wirkstofffreisetzungsrate die Forderung, daß sie eine mit einfachen und kostengünstigen Mitteln beliebig modulierbare Freisetzungskinetik aufweisen kann. Dies wird durch ein beliebiges Mischungsverhältnis der unterschiedlichen Gruppen von Partikeln problemlos erreicht.

Ausgestaltungen der Erfindung sind entsprechend den Unteransprüchen vorgesehen.
Verschiedene Partikel können unterschiedliche Wirkstoffe enthalten. Es können aber auch verschiedene Partikel Wirkstoffe mit unterschiedlichen Konzentrationen enthalten. Weiterhin können Kerne verschiedener Partikel mit unterschiedlicher Größe bzw. Masse und/oder Zusammensetzung ihrer wirkstoffhaltigen Substanz ausgebildet sein. Darüber hinaus kann es besonders vorteilhaft sein, daß die wirkstoffhaltigen Kerne im Gastrointestinaltrakt löslich oder unlöslich, erodierbar oder weitgehend nicht erodierbar sind.

Trägersubstrat: Hierunter ist eine Substanz zu verstehen, die zur Aufnahme von einer ersten Gruppe von Partikeln und mindestens einer weiteren Gruppe von Partikeln befähigt ist. Man kann sie in bestimmten Ausführungsformen der Erfindung verwenden, wenn z.B. eine makroskopisch gesehen homogene Masse hergestellt werden soll.

Die Erfindung sieht auch vor, daß die im Trägersubstrat enthaltenen Partikel eine Mischung von Einheiten gleichen Typs sind. Dies schließt aber nicht aus, daß die im Trägersubstrat enthaltenen Partikel auch eine Mischung von Einheiten unterschiedlichen Typs sein können.

Unter Trägersubstrat wird ein fester oder fließfähiger Stoff oder Stoffgemisch bezeichnet, welcher das Partikelgemisch beinhaltet. Derartige Stoffe, die physiologisch unbedenklich sein müssen, sind dem Fachmann grundsätzlich bekannt. Je nach Anforderung werden besonders für die rektale bzw. vaginale Applikationsform schnell schmelzende Stoffe verwendet. Beispielsweise kommen hierfür Fette wie Kakaofett, Hartfette (Witepsol®, Estarinum®, Novata®), Macrogole und Gelatinen zur Anwendung. Darüber hinaus kann das Trägersubstrat eine weitere stabilisierende Umhüllung umgeben. Diese Umhüllung kann beispielsweise durch Hart- bzw. Weichgelatinekapseln realisiert werden. Diese Umhüllung, die nicht mit der den Kern der Partikel umhüllenden Mantelschicht verwechselt werden darf, hat lediglich die Funktion, gegebenenfalls die Zubereitung mit oder ohne Trägersubstrat aufzunehmen. Die Erfindung schließt nicht aus, daß eine solche, vor Applikation weitgehende formstabile Kapsel ausschließlich das Partikelgemisch enthält.

Freisetzung: Freisetzung nennt man die Abgabe des oder der im Kern der Partikel enthaltenen Wirkstoff(e) in das sie Partikel umgebende Medium. Eine kontrollierte Freisetzung wird erfindungsgemäß erzielt durch die Verwendung von einer ersten Gruppe von Partikeln und mindestens einer weiteren Gruppe von Partikeln, die mit der ersten Gruppe von Partikeln nicht identisch ist. Da jede dieser nicht identischen Gruppen ihr eigenes charakteristisches Freisetzungsverhalten für den oder die Wirkstoffe zeigt, entsteht so bei gleichzeitiger Verabreichung dieser nicht identischen Gruppen eine Überlagerung von nicht identischem Freisetzungsverhalten.

Mischungsverhältnis: Das Mischungsverhältnis bezieht sich auf die Zusammensetzung der Zubereitung und zwar konkret auf die mengenmäßige Zusammensetzung der in der Zubereitung enthaltenen Zahl von Gruppen von Partikeln. Grundsätzlich existieren keine Einschränkungen hinsichtlich möglicher Mischungsverhältnisse. Zweckmäßigerweise (z.B. bedingt durch das Erfordernis möglichst geringer Herstellungskosten) ist die Zahl der Gruppen von Partikeln in der Zubereitung möglichst klein zu halten. Bedingt durch spezielle Therapieerfordernisse (z.B. mehrere Wirkstoffe; sehr spezielle, z.B. gepulste Freisetzung) kann die Zahl der Gruppen von Partikeln jedoch auch verhältnismäßig hoch sein. Bevorzugt sind daher Zahlen von Gruppen von Partikeln, die bei 2, 3, 4 oder 5 liegen. Natürlich können aber auch bis zu zehn oder mehr Gruppen von Partikeln in der Zubereitung enthalten sein. Es sind letztendlich die Erfordernisse der jeweiligen Anwendung, die das konkrete Mischungsverhältnis der verschiedenen Gruppen von Partikeln in der Zubereitung bestimmen.
Gegenstand der Erfindung ist die Verwendung einer Zubereitung, enthaltend eine erste Gruppe von Partikeln und mindestens eine weitere Gruppe von Partikeln, die nicht identisch mit der ersten Gruppe von Partikeln ist, zur Erzielung einer kontrollierten Freisetzung eines in den Kernen der Partikel enthaltenen Wirkstoffs, insbesondere im humanund tiermedizinischen Bereich.

Die Zubereitung wird oral, rektal, vaginal etc. appliziert. Die Freisetzung des Wirkstoffs erfolgt dann in dem entsprechenden Medium, die Resorption des Wirkstoffs erfolgt transmucosal.

Eine Verwendung der Zubereitung nach der Erfindung ist zur steuerbaren Freisetzung von Wirkstoff im Magensaftbereich vorgesehen. Sie kann jedoch auch zur steuerbaren Freisetzung von Wirkstoff im Gastrointestinaltrakt, insbesondere im Dünndarm vorgesehen sein. Ein solcher Unterschied ergibt sich in an sich bekannter Art abhängig vom pH-Wert der Körperflüssigkeit, einerseits im sauren Bereich des Magens, oder andererseits im neutralen oder basischen Bereich des Dünndarms. Vorzugsweise dient die Zubereitung zur beliebig modulierbaren Steuerung und insbesondere zur linearen Steuerung der Wirkstofffreisetzung. Schließlich kann die Zubereitung zur steuerbaren Freisetzung von Wirkstoff, beispielsweise in Form eines Formlings wie Zäpfchen im Anal- oder Vaginalbereich, Verwendung finden.

Kernsubstanz: Diese Substanz besitzt die Funktion, ein Trägermaterial für den im Kern enthaltenen Wirkstoff zu sein. Auf die Verwendung einer Kernsubstanz kann verzichtet werden, falls die Eigenschaften des Wirkstoffs die Verwendung eines Trägermaterials überflüssig machen oder falls der Wirkstoff bzw. die Wirkstoffe in reiner Form verwendet werden sollen.

Die Kernsubstanz der einzelnen Partikel enthält in der Regel neben einem oder mehreren Wirkstoffen und einer Trägersubstanz als Füllstoff noch weitere Hilfsstoffe als Zusätze. Die erwähnten Zusätze - auch Hilfsstoffe genannt - werden nach ihrer Funktion in Weichmacher, Klebrigmacher, Resorptionsvermittler, Stabilisatoren oder Fließregulierungsmittel eingeteilt. Derartige Stoffe, die physiologisch unbedeklich sein müssen, sind dem Fachmann grundsätzlich bekannt. Als Trägersubstanz oder Füllstoffe werden Stoffe eingesetzt, welche den Anforderungen entsprechende Löslichkeits-, Erodier- bzw. Quelleigenschaften aufweisen. Typische Substanzen sind hierfür beispielsweise Laktose, Cellulosearten, Zuckeralkohole wie z.B. Manitol und Sorbitol, verschiedene Stärken und Alginate.

Mantelsubstanz (Umhüllungssubstanz): Hierbei handelt es sich um erodierbares Material. Dieses Material ist in der Lage, bei Kontakt mit einem Medium seine strukturelle Integrität zu verlieren. Als Medium können z.B. Wasser, Magensaft, Säuren, weitere Flüssigkeiten dienen. Darunter kann man aber auch einen Raum mit gegenüber Normalbedingungen veränderten Parametern verstehen, wie z.B. einen Raum mit erhöhter Temperatur und/oder erhöhter Luftfeuchtigkeit (wie die menschliche Lunge). Mit dem Verlust der strukturellen Integrität ist gemeint, daß diese Substanz ihre äußere und/oder innere Struktur verliert, z.B. durch physikalische und/oder chemische Umwandlung. Damit sind Prozesse wie Schmelzen, Lösen, Quellen und Zersetzung gemeint. Stoffe, die bei Kontakt mit einem der genannten Arten von Medium erodieren können, können monomere und/oder polymere Substanzen und/oder deren Mischungen sein.

Zu geeigneten monomeren Mantelsubstanzen zählen Wachs, Salze wie NaCl, Zucker wie Saccararose und andere Stoffe. Zu den geeigneten polymeren Mantelsubstanzen zählen Magensaft oder im Dünndarm lösliche Substanzen.

Ein weiteres Merkmal der Mantelsubstanz ist die Eigenschaft, daß eine Diffusion des Wirkstoffs bzw. der Wirkstoffe durch die Mantelsubstanz praktisch nicht stattfindet. Damit ist gemeint, daß die Mantelsubstanz für die Wirkstoffe nicht passierbar ist oder daß die Diffusion des oder der Wirkstoffe durch die Mantelschicht einen längeren Zeitraum erfordert als der Zeitraum, der zwischen Herstellung der Partikel und ihrer Anwendung liegt.

Die Kernsubstanz eines Partikels kann gegebenenfalls mit der Mantelsubstanz dieses Partikels identisch sein; es kann sich aber auch um unterschiedliche Materialien handeln. Sofern Kernsubstanz und Mantelsubstanz identisch sind, kann man den Kern durch die Existent des Wirkstoffs oder der Wirkstoffe von der Mantelschicht unterscheiden.

Als magensaftlösliche Mantelsubstanz werden beispielsweise Acrylsäurederivate wie Eudragit®L bzw. Eudragit®S, Polyvinylalkohole, Hydroxypropylmethylcelluloseacetatphthalat oder Celluloseacetatphthalat, als im Dünndarm lösliche Substanzen Polyacrylsäure-ester wie beispielsweise Eudragit®E30D, Polyacrylsäurederivate, Schellack oder Cellulose wie Methyl- bzw. Ethylcellulose eingesetzt.

Wirkstoffe sind Stoffe, die eine chemische oder pharmazeutische Wirkung ausüben können. Hierzu zählen niedermolekulare und hochmolekulare Stoffe. Solche Stoffe sind dem Fachmann bekannt. Zu den hochmolekularen Stoffen zählen unter anderem Enzyme, Impfstoffe, Insulin, Peptide, Proteine und Biopolymere.

Geeignete Wirkstoffe finden sich in den Wirkstoffgruppen der Parasympatholytika (z.B. Scopolamin, Atropin, Berlactyzin), der Cholinergika (z.B. Physostigmin, Nicotin), der Neuroleptika (z.B. Chlorpromazin, Haloperidol), der Monoaminoxidasehemmer (z.B. Tranylcypromin, Selegilin), der Sympathomimetika (z.B. Ephedrin, D-Norpseudoephedrin, Salbutamol, Fenfluramin), der Sympatholytika und Antisympathotonika (z.B. Propanolol, Timolol, Bupranolol, Clonidin, Dihydroergotamin, Naphazolin), der Anxiolytika (z.B. Diazepam, Triazolam), der Lokalanästhetika (z.B. Lidocain), der zentralen Analgetika (z.B. Fentanyl, Sufentanil), der Antirheumatika (z.B. Indomethacin, Piroxicam, Lornoxicam), der Koronartherapeutika (z.B. Glyceroltrinitrat, Isosorbiddinitrat), der Östrogene, Gestagene und Androgene, der Antihistaminika (z.B. Diphenhydramin, Clemastin, Terfenadin), der Prostaglandinderivate, der Vitamine (z.B. Vitamin E, Cholecalciferol) und der Cytostatika.

Weitere Einzelheiten, Merkmale und Vorteile der Erfindung ergeben sich aus der nachstehenden Erläuterung eines in den Zeichnungen schematisch dargestellten Ausführungsbeispieles. Es zeigen:
- Figur 1:: ein Trägersubstrat 1 mit eingelagerten unterschiedlichen Partikeln 2 mit identischer Kernsubstanz 3, gleichem Wirkstoff A, identischer Mantelsubstanz und unterschiedlicher Manteldicke 4,
- Figur 2:: ein Trägersubstrat 1 mit eingelagerten unterschiedlichen Partikeln 2 mit identischer Kernsubstanz 3, unterschiedlichen Wirkstoffen A-C, identischer Mantelsubstanz und unterschiedlicher Manteldicke 4,
- Figur 3:: ein Trägersubstrat 1 mit eingelagerten unterschiedlichen Partikeln 2 mit identischer Kernsubstanz 3, mehreren Wirkstoffen A-D je Partikelkern, identischer Mantelsubstanz und unterschiedlicher Manteldicke 4,
- Figur 4:: ein Trägersubstrat 1 mit eingelagerten unterschiedlichen Partikeln 2 mit unterschiedlicher Kernsubstanz 3 und unterschiedlichem Kernradius, gleichem Wirkstoff A, identischer Mantelsubstanz und unterschiedlicher Manteldicke 4,
- Figur 5:: ein Trägersubstrat 1 mit eingelagerten unterschiedlichen Partikeln 2 mit identischer Kernsubstanz 3, gleichem Wirkstoff A, unterschiedlicher Mantelsubstanz und Manteldicke 4,
- Figur 6:: ein Diagramm eines Freisetzungsprofils von Wirkstoff in Abhängigkeit von der Zeit.

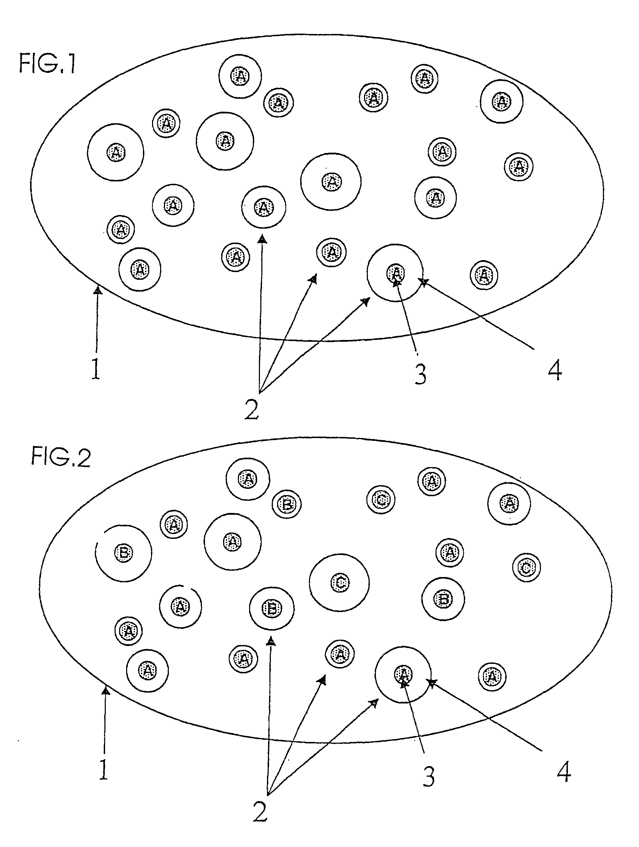

Die in Figur 1 dargestellte orale oder mucosale Zubereitung mit steuerbarer Wirkstofffreisetzungsrate weist ein Trägersubstrat 1 mit eingelagerten Partikeln 2, bestehend aus einem Kern 3 und einer wirkstofffreien Umhüllung 4 auf. Erfindungsgemäß ist die wirkstofffreie Umhüllung im feuchten bzw. wäßrigen Milieu einer Körperflüssigkeit erodierbar. Dabei kann es sich um jede Art von Flüssigkeit handeln wie beispielsweise Magen- oder Intestinalflüssigkeit, Flüssigkeiten der Mundhöhle oder auch anderer Körperhöhlen, beispielsweise im Anal- oder Vaginalbereich. Wesentlich ist weiterhin, daß das Trägersubstrat 1 eine in feuchtem bzw. wäßrigen Milieu einer Körperflüssigkeit rasch zerfallende und die Partikel 2 freisetzende Zubereitung ist.
Dabei können Partikel 2 mit gleicher Kernsubstanz und gleicher Umhüllungssubstanz abhängig von deren relativen Gewichtsanteilen unterschiedlichen Gruppen zugeordnet werden, wobei gleiche Gruppen ein gleiches Erosions- und Freisetzungsverhalten aufweisen.
Das Mittel zur Steuerung der Freisetzungskinetik des Wirkstoffs ist dann das Mischungsverhältnis unterschiedlicher Gruppen von Partikeln 2.

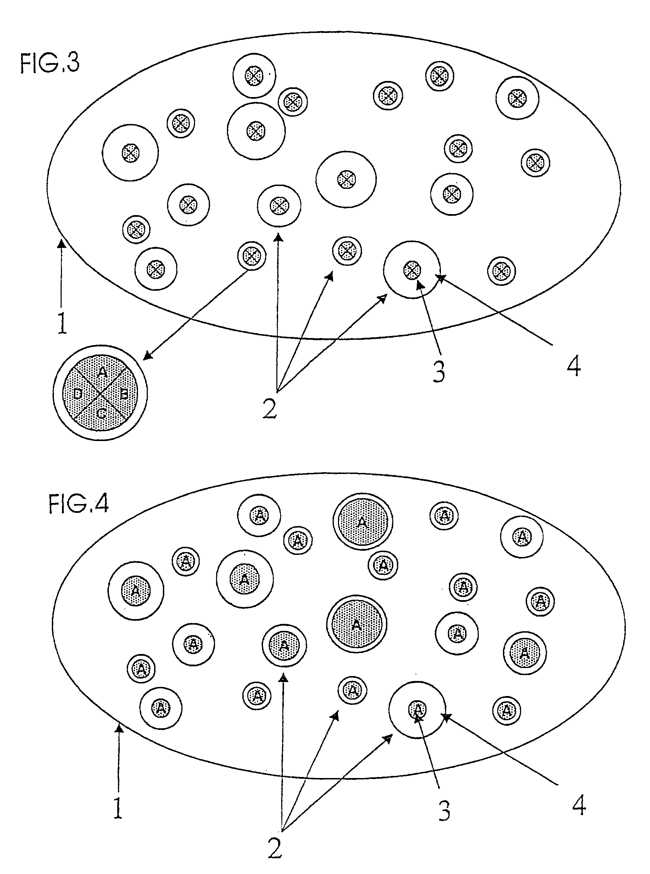

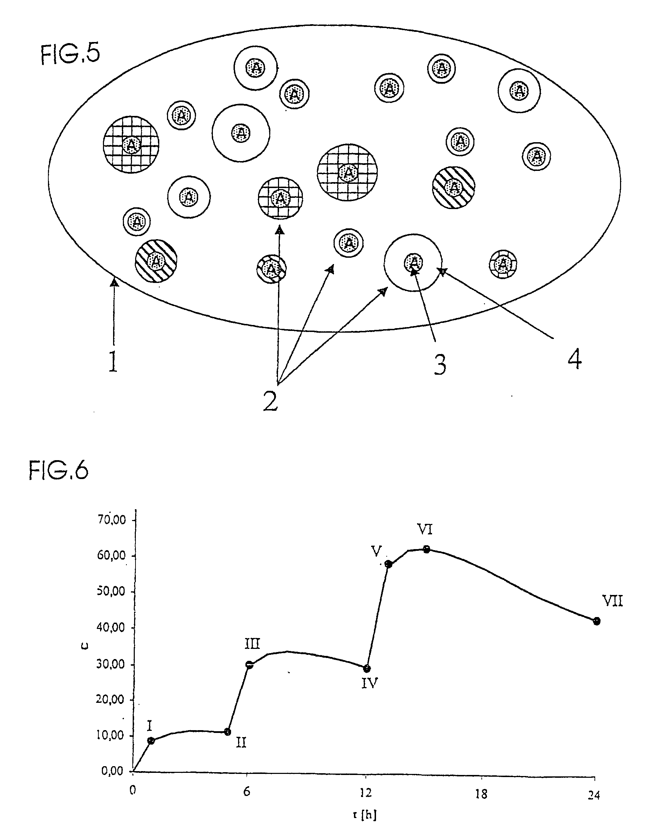

Die Figuren 1 bis 5 zeigen hierzu verschiedene Ausprägungsformen der Erfindung. Wie dazu Figur 1 zeigt, können Kerne 3 verschiedener Partikel 2 identischen Wirkstoff (A) enthalten, wobei dieser Wirkstoff in unterschiedlichen Konzentrationen vorliegen kann. In Figur 2 enthalten Kerne 2 verschiedener Partikel 3 unterschiedliche Wirkstoffe (A, B, C). Figur 3 zeigt eine Ausprägungsform der Erfindung in welcher mehrere Wirkstoffe (A-D) in den Kernen 2 der Partikel 3 enthalten sind, wobei das Wirkstoffgemisch verschiedener Kerne 2 unterschiedlich sein kann. Wie Figur 4 zeigt, können darüber hinaus Kerne 3 verschiedener Partikel 2 mit unterschiedlicher Größe bzw. Masse und/oder Zusammensetzung ihrer wirkstoffhaltigen Substanz ausgebildet sein. Weiterhin kann die Dicke und/oder stoffliche Art bzw. Zusammensetzung der Umhüllung 4 einzelner Partikel 2 unterschiedlich sein (Figur 5).
Wie Figur 1 weiter zeigt, können die im Trägersubstrat 1 enthaltenen Partikel 2 eine Mischung von Einheiten gleichen Typs oder unterschiedlichen Typs sein.

Figur 6 zeigt ein Freisetzungsprofil von Wirkstoff im Verlauf einer sich über längere Zeit erstreckenden Wirkstofffreigabe. Dazu ist auf der Abszisse die Zeit zwischen 0 und 24 h und auf der Ordinate die Abgabemenge von Wirkstoff in einer Skala zwischen 0 und 70 (beispielsweise µg) angegeben.
Dabei zeigt der Verlauf des Diagramms zwischen 0 und I einen vergleichsweise steilen, jedoch linearen Anstieg der Wirkstoffabgabe nach Freilegung eines wirkstoffhaltigen Kerns 3 infolge vorausgegangener Erosion einer wirkstofffreien Umhüllung 4. Der weitere Verlauf der Diagrammkurve zwischen I und II zeigt dann eine Erschöpfung des Wirkstoffgehaltes einer ersten Partikelgruppe, wonach etwa nach 5 ½ h die Umhüllungen einer zweiten Gruppe erodiert sind und zwischen den Kurvenpunkten II und III wiederum ein starker, linearer Anstieg der Kurve zu verzeichnen ist, bis auch zwischen 6 und 12 h, entsprechend den Kurvenästen III und IV der Wirkstoffgehalt der freigelegten Kerne 3 verbraucht ist und der Wirkstoffgehalt im Körper wieder langsam abnimmt, bis bei Beginn der zwölften Stunde eine weitere Gruppe von Partikeln nach Erodieren der Kernumhüllungen die Wirkstoffabgabe in steilem Kurvenverlauf von IV zu V fortsetzt, wobei im Punkt VI die höchste Wirkstoffkonzentration im Körper mit ca. 65 µg erreicht ist. Nach Erschöpfung des Wirkstoffgehaltes der letzten Gruppe sinkt dann während der folgenden etwa 8 h der Wirkstoffgehalt im Körper zwischen den Punkten VI und VII von ca. 65 µg auf ca. 40 µg ab.

Die Herstellung der erfindungsgemäßen Zubereitung kann durch Verfahren erfolgen, die Kombinationen der folgenden, dem Fachmann einzeln bekannten Schritte darstellen können:
1) Herstellen der wirkstoffhaltigen Kerne der ersten Gruppe von Partikeln,
2) Herstellen der wirkstoffhaltigen Kerne einer zweiten Gruppe von Partikeln,
3) Ummanteln der wirkstoffhaltigen Kerne der ersten Gruppe von Partikeln mit einer ersten Mantelschicht,
4) Ummanteln der wirkstoffhaltigen Kerne der zweiten Gruppe von Partikeln mit einer zweiten Mantelschicht, wobei entweder die Kerne der ersten Gruppe von Partikeln nicht identisch sind mit den Kernen der zweiten Gruppe von Partikeln oder die erste Mantelschicht nicht identisch ist mit der zweiten Mantelschicht,
5) Mischen der ersten Gruppe von Partikeln und der zweiten Gruppe von Partikeln.

Die zeitliche Reihenfolge dieser Schritte ist insofern eingeschränkt, als das Mischen (Schritt 5) erst nach Herstellung der beiden Gruppen von Partikeln erfolgen kann.

Die Herstellung der wirkstoffhaltigen Kerne kann in einem einzigen Schritt erfolgen, wenn sich die erste Gruppe von Partikeln nur durch unterschiedliche Mantelschichten von der zweiten Gruppe von Partikeln unterscheidet. Ebenso kann die Herstellung der Mantelschichten in einem einzigen Schritt erfolgen, sofern sich die erste Gruppe von Partikeln nur durch unterschiedliche wirkstoffhaltige Kerne von der zweiten Gruppe von Partikeln unterscheidet.

Das Herstellen der Kerne kann z.B. in einer Kugelmühle erfolgen, das Herstellen einer Mantelschicht kann z.B. in einem Sprühbeschichter erfolgen. Für das Ummanteln von Kernen mit einer Größe unterhalb von 100 µm mit Mantelschichten mit einer Schichtdicke zwischen < 1 µm bis zu wenigen µm kann man z.B. das in DE 197 11 393 C1 beschriebene Verfahren verwenden.

Die Erfindung ist unkompliziert und ermöglicht mit einfachen Mitteln eine beliebige Steuerung der Freisetzungskinetik des Wirkstoffs in Abhängigkeit von Mischungsverhältnis unterschiedlicher Gruppen von Partikeln und löst damit in optimaler Weise die eingangs gestellte Aufgabe.

## Patentansprüche

1. Wirkstoff enthaltende orale, rektale oder vaginale Zubereitung mit steuerbarer Wirkstofffreisetzung zur transmucosalen Resorption des Wirkstoffs, die unter Ausbildung eines multipartikulären Systems eine Vielzahl von Partikeln (2) enthält, wobei jedes Partikel (2) aus einem wirkstoffhaltigen Kern (3) und einer im feuchten bzw. wäßrigen Milieu einer Körperflüssigkeit erodierbaren bzw. löslichen, weitgehend wirkstofffreien Umhüllung (4) besteht, und wobei das multipartikuläre System die Partikel (2) an bzw. in einem Trägersubstrat (1) enthält und das Trägersubstrat (1) eine in feuchtem bzw. wäßrigem Milieu einer Körperflüssigkeit rasch zerfallende und die Partikel (2) freisetzende Zubereitung ist, **dadurch gekennzeichnet,**
- **daß** Partikel (2) mit gleicher Kernsubstanz und gleicher Umhüllungssubstanz entsprechend deren relativen Gewichtsanteilen unterschiedlichen Gruppen zuzuordnen sind, wobei gleiche Gruppen ein gleiches Erosions- und Freisetzungsverhalten aufweisen, und
- **daß** das Mittel zur Steuerung der Freisetzungskinetik des Wirkstoffs das Mischungsverhältnis unterschiedlicher Gruppen von Partikeln (2) ist.

2. Zubereitung nach Anspruch 1, **dadurch gekennzeichnet, daß** Kerne (3) verschiedener Partikel (2) unterschiedliche Wirkstoffe enthalten.

3. Zubereitung nach Anspruch 1 oder 2, **dadurch gekennzeichnet, daß** Kerne (3) mehrere Wirkstoffe enthalten.

4. Zubereitung nach einem oder mehreren der Ansprüche 1 bis 3, **dadurch gekennzeichnet, daß** Kerne (3) verschiedener Partikel (2) mit unterschiedlicher Größe bzw. Masse und/oder Zusammensetzung ihrer wirkstoffhaltigen Substanz ausgebildet sind.

5. Zubereitung nach einem oder mehreren der Ansprüche 1 bis 4, **dadurch gekennzeichnet, daß** die im Trägersubstrat (1) enthaltenen Partikel (2) eine Mischung von Einheiten gleichen Typs sind.

6. Zubereitung nach einem oder mehreren der Ansprüche 1 bis 5, **dadurch gekennzeichnet, daß** die im Trägersubstrat (1) enthaltenen Partikel (2) eine Mischung von Einheiten unterschiedlichen Typs sind.

7. Zubereitung nach einem oder mehreren der Ansprüche 1 bis 6, **dadurch gekennzeichnet, daß** Kerne (3) verschiedener Partikel (2) im sauren und/oder basischen Bereich des Gastrointestinaltraktes weitgehend unlöslich sind.

8. Zubereitung nach einem oder mehreren der Ansprüche 1 bis 7, **dadurch gekennzeichnet, daß** Kerne (3) verschiedener Partikel (2) im sauren und/oder basischen und/oder neutralen Bereich des Gastrointestinaltraktes löslich sind, wobei sich die Löslichkeit der Kerne von der Löslichkeit ihrer Umhüllung um mindestens 10 % unterscheidet.

9. Zubereitung nach einem oder mehreren der vorangehenden Ansprüche, enthaltend eine erste Gruppe von untereinander identischen Partikeln und mindestens eine weitere Gruppe von untereinander identischen Partikeln, **dadurch gekennzeichnet, daß**
a) die Partikel der ersten Gruppe einen Kern und eine Mantelschicht besitzen,
b) die Partikel der mindestens einen weiteren Gruppe einen Kern und eine Mantelschicht besitzen,
c) die untereinander identischen Partikel der ersten Gruppe nicht identisch sind mit den untereinander identischen Partikeln der mindestens einen weiteren Gruppe,
d) der Kern der Partikel der ersten Gruppe ein bestimmtes Gewicht GK1 besitzt,
e) die Mantelschicht der Partikel der ersten Gruppe ein bestimmtes Gewicht GU1 besitzt,
f) die besagten Gewichte von Kern und Mantelschicht der Partikel der ersten Gruppe in einem bestimmten, konstanten Verhältnis GK1 : GU1 zueinander stehen,
g) der Kern der Partikel der mindestens einen weiteren Gruppe ein bestimmtes Gewicht GKn besitzt,
h) die Mantelschicht der Partikel der mindestens einen weiteren Gruppe ein bestimmtes Gewicht GUn besitzt,
i) die besagten Gewichte von Kern und Mantelschicht der Partikel der mindestens einen weiteren Gruppe in einem bestimmten, konstanten Verhältnis GKn : GUn zueinander stehen, und
das Verhältnis GK1 : GU1 nicht identisch ist mit dem Verhältnis GKn : GUn.

10. Zubereitung nach Anspruch 9, **dadurch gekennzeichnet, daß**
a) der im Kern der Partikel der ersten Gruppe enthaltene Wirkstoff und der im Kern der Partikel der mindestens einen weiteren Gruppe enthaltene Wirkstoff identisch sind, und
b) die Mantelschicht der Partikel der ersten Gruppe und die Mantelschicht der mindestens einen weiteren Gruppe nicht identisch sind.

11. Zubereitung nach Anspruch 9, **dadurch gekennzeichnet, daß** der im Kern der Partikel der ersten Gruppe enthaltene Wirkstoff und der im Kern der Partikel der mindestens einen weiteren Gruppe enthaltene Wirkstoff nicht identisch sind.

12. Verfahren zur Herstellung einer Zubereitung nach einem oder mehreren der vorangehenden Ansprüche, enthaltend eine erste Gruppe von untereinander identischen Partikeln und mindestens eine weitere Gruppe von untereinander identischen Partikeln, wobei die untereinander identischen Partikel der ersten Gruppe nicht identisch sind mit den untereinander identischen Partikeln der mindestens einen weiteren Gruppe, **gekennzeichnet durch** die Schritte:
a) Herstellen der wirkstoffhaltigen Kerne der ersten Gruppe von Partikeln,
b) Herstellen der wirkstoffhaltigen Kerne einer zweiten Gruppe von Partikeln,
c) Ummanteln der wirkstoffhaltigen Kerne der ersten Gruppe von Partikeln mit einer ersten Mantelschicht,
d) Ummanteln der wirkstoffhaltigen Kerne der zweiten Gruppe von Partikeln mit einer zweiten Mantelschicht, wobei entweder die Kerne der ersten Gruppe von Partikeln nicht identisch sind mit den Kernen der zweiten Gruppe von Partikeln oder die erste Mantelschicht nicht identisch ist mit der zweiten Mantelschicht, und
e) Mischen der ersten Gruppe von Partikeln und der zweiten Gruppe von Partikeln.

## Claims

1. An active ingredient-containing oral, rectal or vaginal preparation for transmucosal absorption of the active ingredient, with controllable active ingredient release, which contains a plurality of particles (2) to form a multiparticulate system, each particle (2) consisting of an active ingredient-containing core (3) and of a covering (4) which is substantially free of active ingredient and is erodible or soluble in the moist or aqueous medium of a body fluid, and the multiparticulate system containing the particles (2) on or in a carrier substrate (1), and the carrier substrate (1) being a preparation which rapidly disintegrates in the moist or aqueous medium of a body fluid and releases the particles (2), **characterized**
- **in that** particles (2) with identical core substance and identical covering substance are to be assigned to different groups according to their relative proportions by weight, with identical groups displaying identical erosion and release behaviour, and
- **in that** the means for controlling the release kinetics of the active ingredient is the mixing ratio of different groups of particles (2).

2. A preparation according to Claim 1, **characterized in that** cores (3) of different particles (2) contain different active ingredients.

3. A preparation according to Claim 1 or 2, **characterized in that** cores (3) contain a plurality of active ingredients.

4. A preparation according to one or more of Claims 1 to 3, **characterized in that** cores (3) of different particles (2) differ in size or mass and/or composition of their active ingredient-containing substance.

5. A preparation according to one or more of Claims 1 to 4, **characterized in that** the particles (2) present in the carrier substrate (1) are a mixture of units of identical type.

6. A preparation according to one or more of Claims 1 to 5, **characterized in that** the particles (2) present in the carrier substrate (1) are a mixture of units of different types.

7. A preparation according to one or more of Claims 1 to 6, **characterized in that** cores (3) of different particles (2) are substantially insoluble in the acidic and/or basic region of the gastrointestinal tract.

8. A preparation according to one or more of Claims 1 to 7, **characterized in that** cores (3) of different particles (2) are soluble in the acidic and/or basic and/or neutral region of the gastrointestinal tract, with the solubility of the cores differing from the solubility of their covering by at least 10%.

9. A preparation according to one or more of the preceding claims, comprising a first group of mutually identical particles and at least one other group of mutually identical particles, **characterized in that**
a) the particles of the first group have a core and a coating layer,
b) the particles of the at least one other group have a core and a coating layer,
c) the mutually identical particles of the first group are not identical to the mutually identical particles of the at least one other group,
d) the core of the particles of the first group has a particular weight GK1,
e) the coating layer of the particles of the first group has a particular weight GU1,
f) said weights of core and coating layer of the particles of the first group are in a particular constant ratio GK1:GU1,
g) the core of the particles of the at least one other group has a particular weight GKn,
h) the coating layer of the particles of the at least one other group has a particular weight GUn,
i) the said weights of core and coating layer of the particles of the at least one other group are in a particular constant ratio GKn:GUn, and
the ratio GK1:GU1 is not identical to the ratio GKn:GUn.

10. A preparation according to Claim 9, **characterized in that**
a) the active ingredient present in the core of the particles of the first group and the active ingredient present in the core of the particles of the at least one other group are identical, and
b) the coating layer of the particles of the first group and the coating layer of the at least one other group are not identical.

11. A preparation according to Claim 9, **characterized in that** the active ingredient present in the core of the particles of the first group and the active ingredient present in the core of the particles of the at least one other group are not identical.

12. A process for the production of a preparation according to one or more of the preceding claims, comprising a first group of mutually identical particles and at least one other group of mutually identical particles, where the mutually identical particles of the first group are not identical to the mutually identical particles of the at least one other group, **characterized by** the steps:
a) production of the active ingredient-containing cores of the first group of particles,
b) production of the active ingredient-containing cores of a second group of particles,
c) coating of the active ingredient-containing cores of the first group of particles with a first coating layer,
d) coating of the active ingredient-containing cores of the second group of particles with a second coating layer, where either the cores of the first group of particles are not identical to the cores of the second group of particles, or the first coating layer is not identical to the second coating layer, and
e) mixing the first group of particles and the second group of particles.

## Revendications

1. Composition orale, rectale ou vaginale contenant une ou plusieurs substances actives, destinée à la résorption transmuqueuse de la ou des substances actives avec une libération de substance active réglable qui contient, en formant un système multiparticulaire, une multitude de particules (2), chaque particule (2) étant constituée d'un noyau (3), contenant une ou plusieurs substances actives, et d'un enrobage (4), dans une large mesure exempt de substance active, pouvant être érodé ou soluble dans le milieu humide ou aqueux d'un liquide corporel et le système multiparticulaire contenant les particules (2) sur ou dans un substrat support (1) et le substrat support (1) étant une composition se décomposant rapidement dans le milieu humide ou aqueux d'un liquide corporel et libérant les particules (2), **caractérisée**
- **en ce que** des particules (2) présentant la même substance dans le noyau et la même substance d'enrobage sont attribuées, en fonction de leurs proportions pondérales relatives, à différents groupes, des groupes identiques présentant un même comportement d'érosion et de libération, et
- **en ce que** le moyen pour régler la cinétique de libération de la substance active est le rapport de mélange des différents groupes de particules (2).

2. Composition selon la revendication 1, **caractérisée en ce que** les noyaux (3) de particules différentes (2) contiennent différentes substances actives.

3. Composition selon la revendication 1 ou 2, **caractérisée en ce que** les noyaux (3) contiennent plusieurs substances actives.

4. Composition selon l'une ou plusieurs des revendications 1 à 3, **caractérisée en ce que** les noyaux (3) de particules différentes (2) sont formés avec une taille ou une masse différente et/ou une composition différente de leur substance contenant la ou les substances actives.

5. Composition selon l'une ou plusieurs des revendications 1 à 4, **caractérisée en ce que** les particules (2) contenues dans le substrat support (1) sont un mélange d'unités de type identique.

6. Composition selon l'une ou plusieurs des revendications 1 à 5, **caractérisée en ce que** les particules (2) contenues dans le substrat support (1) sont un mélange d'unités de types différents.

7. Composition selon l'une ou plusieurs des revendications 1 à 6, **caractérisée en ce que** les noyaux (3) de particules différentes (2) sont dans une large mesure insolubles dans la zone acide et/ou basique du tractus gastro-intestinal.

8. Composition selon l'une ou plusieurs des revendications 1 à 7, **caractérisée en ce que** les noyaux (3) de particules différentes (2) sont solubles dans la zone acide et/ou basique et/ou neutre du tractus intestinal, la solubilité des noyaux se distinguant d'au moins 10% de la solubilité de leur enrobage.

9. Composition selon l'une ou plusieurs des revendications précédentes, contenant un premier groupe de particules identiques les unes aux autres et au moins un autre groupe de particules identiques les unes aux autres,
**caractérisée en ce que**
a) les particules du premier groupe présentent un noyau et une couche d'enrobage,
b) les particules dudit au moins un autre groupe présentent un noyau et une couche d'enrobage,
c) les particules du premier groupe, identiques les unes aux autres, ne sont pas identiques aux particules identiques les unes aux autres dudit au moins un autre groupe,
d) le noyau des particules du premier groupe présente un poids déterminé GK1,
e) la couche d'enrobage des particules du premier groupe présente un poids déterminé GU1,
f) lesdits poids du noyau et de la couche d'enrobage du premier groupe se trouvent dans un rapport déterminé constant GK1:GU1,
g) le noyau des particules dudit au moins un autre groupe présente un poids déterminé GKn,
h) la couche d'enrobage des particules dudit au moins un autre groupe présente un poids déterminé GUn,
i) lesdits poids du noyau et de la couche d'enrobage des particules dudit au moins un autre groupe se trouvent dans un rapport déterminé constant GKn:GUn et
GK1:GU1 n'est pas identique au rapport GKn:GUn.

10. Composition selon la revendication 9, **caractérisée en ce que**
a) la substance active contenue dans le noyau des particules du premier groupe et la substance active contenue dans le noyau des particules dudit au moins un autre groupe sont identiques et
b) la couche d'enrobage des particules du premier groupe et la couche d'enrobage dudit au moins un autre groupe ne sont pas identiques.

11. Composition selon la revendication 9, **caractérisée en ce que** la substance active contenue dans le noyau des particules du premier groupe et la substance active contenue dans le noyau des particules dudit au moins un autre groupe ne sont pas identiques.

12. Procédé pour préparer une composition selon l'une ou plusieurs des revendications précédentes, contenant un premier groupe de particules identiques les unes aux autres et au moins un autre groupe de particules identiques les unes aux autres, les particules identiques les unes aux autres du premier groupe n'étant pas identiques aux particules identiques les unes aux autres dudit au moins un autre groupe, **caractérisé par** les étapes de
a) préparation des noyaux contenant une ou plusieurs substances actives du premier groupe de particules,
b) préparation des noyaux contenant une ou plusieurs substances actives d'un deuxième groupe de particules,
c) enrobage des noyaux contenant une ou plusieurs substances actives du premier groupe de particules avec une première couche d'enrobage,
d) enrobage des noyaux contenant une ou plusieurs substances actives du deuxième groupe de particules d'une deuxième couche d'enrobage, les noyaux du premier groupe de particules n'étant pas identiques aux noyaux du deuxième groupe de particules ou la première couche d'enrobage n'étant pas identique à la deuxième couche d'enrobage, et
e) mélange du premier groupe de particules et du deuxième groupe de particules.
